# EUROPEAN PATENT APPLICATION

(11) **EP 3 537 445 A1**
(43) Date of publication of application: **11.09.2019**
(21) Application number: 18160412.5
(22) Date of filing: 07.03.2018
(51) Int. Cl.: G16H 20/30

(54) **A SYSTEM FOR TRACKING TASKS PERFORMED BY A USER**

(71) Applicant: Leo Pharma A/S, 2750 Ballerup (DK)
(72) Inventor: CAIRO, Cristiano Riccardo, 2770 Kastrup (DK); JØRGENSEN, Morten Schnack, 2400 Copenhagen NV (DK); BÆRENTZEN, Troels Ravn, 2720 Vanløse (DK)
(74) Representative: Inspicos P/S

(57) **Abstract**

An activity tracking system is configured to track one or more tasks performed by a user. The system comprises at least one smart device, a software platform, and a computer unit wirelessly connected to the at least one smart device. The computer unit is configured to run the software platform and the software platform is configured for exchanging information between the at least one smart device and computer unit. Each smart device may be configured to receive from the computer unit a plan of tasks and send, to the computer unit, a task signal related to a task performed by the user, the task signal being generated when the user press the smart device, and wherein the computer unit is configured to receive the task signal and log the task performed along with time and date of the task.

## Description

### FIELD OF THE INVENTION

The present invention relates to a system for tracking and logging one or more tasks performed by a user.

### BACKGROUND OF THE INVENTION

People usually need reminders to do tasks they are supposed to do. Also, people are often forgetting to take a medication prescribed to them, especially if the treatment is related to a chronical disease. Likewise, children need to be reminded of taking medication. A number of chronical diseases, especially skin diseases such as psoriasis, can be well treated if a prescribed medication is used in accordance to a prescribed schedule. In this respect, there is a need for a system which will motivate a user to create good habits and be able to be aware of his behaviour.

### SUMMARY OF THE INVENTION

On the above backgrounds, it is an object of embodiments of the invention to provide a simple and intuitive click button which would motivate a user to track various tasks diligently and on time. Furthermore, it is an object of the invention to create and improve behaviours and habits of users. In particular, it is an object of embodiments of the invention to provide a simple and unique system facilitating communication and interaction between the user, a user's smart phone and a click button.

In a first aspect, the present invention provides an activity tracking system configured to track one or more tasks performed by a user, the system comprising at least one smart device, a software platform, and a computer unit wirelessly connected to the at least one smart device, the computer unit being configured to run the software platform, the software platform configured for exchanging information between the at least one smart device and the computer unit, wherein
- each smart device is configured to send, to the computer unit, a task signal related to a task performed by the user, the task signal being generated when the user activates the smart device by push, touch or voice activation, and wherein the computer unit is configured to receive the task signal and log the task performed along with time and date of the task, and wherein
- the smart device comprises a printed circuit board (PCB), the PCB comprising a memory medium configured to temporarily store at least one task signal along with time and date of the task in case the computer unit is out of reach.

By tracking activities and tasks, the user can improve and follow own habits. Furthermore, the user can be reminded to perform a planned task on time. The smart device is preferably configured for activation in a simple, easy, and intuitive manner, such as, e.g, by clicking a button, moving the smart device, touching a pad or generating a predetermined acoustic signal. An account may be held by the user, the account being created and accessed using the software platform. Once the account is created, the smart device may connect wirelessly to the computer unit. Then the user may choose one or more tasks which he wants to track, or in relation to which habits need to be improved. The smart device may be removably attached to an object which is in the user's possession, and which may be associated with the performance of a certain task. In the software platform, the user may create a plan that he wants to follow. However, the system may work without any plan, and be used for tracking a certain task or tasks, such as by counting a number of activations of the smart device within predetermined time intervals, or by recording time intervals between activations. The system does not require a constant connection between the smart device and the computer unit, as the smart unit is equipped with a memory medium capable of storing at least one task registration. Preferably, the registered data may be communicated to the computer unit at a later point in time, once a connection has been established.

In the present context, the term "smart device" is to be interpreted as unit which can be activated by a user through mechanical interaction, such as by push-button or touch activation, by voice, or by movement of the device. It may preferably be embodied in the form of a single click button device powered by a long lasting battery, preferably with dimensions not exceeding 4 cm x 4 cm x 1 cm. In a preferred embodiment, the smart device comprises no monitor or display. Preferably, it further comprises no keyboard or similar user interface other than a push-button, touch or voice interface for user activation, and possibility to easily move it. Preferred embodiments of the smart device are simple and intuitive to start and operate. At the very first use of embodiments of the smart device, the user simply may be required to click the button, and the smart device will be turned on and will be immediately ready to be wirelessly connected to the computer unit. No particular instructions may be needed for setting up and/or operating the smart device and therefore there is no need for a special education of the potential users. Further, use of preferred embodiments the smart device is also preferably simple - the only thing the user has to perform is to tap, click, move, touch or press the button, or generate a predetermined sound for activation. Therefore, the preferred embodiments of the smart device are usable by both children and elderly people.

Embodiments of the smart device may be configured to store, in the memory medium, a plan to perform alerts, the plan being preferably downloaded from the computer unit. Thus, alerts may be generated when the computer unit and smart device are out of each other's reach.

The smart device comprises the printed circuit board (PCB) which preferably carries the majority of electronics necessary for operating the smart device, such as the memory medium, means for wirelessly connecting to the computer unit such as, e.g., a Bluetooth antenna, and a power source, such as a battery. The function of the memory medium during time periods when the smart device is turned on and activated by the user but not connected to the computer unit is to temporarily store task signals and their time stamps until a wireless connection to the computer unit is established. The smart device may not be connected to the computer unit for various reasons, e.g., the computer unit may be out or reach or it may be turned off. Additionally, the PCB may comprise a switch, touch sensor, acoustic sensor, and/or an indicator which may indicate that the smart device is being activated either by a physical interaction (e.g. press, move, touch, etc.) or by a voice command. The indicator may be a light emitting diode (LED), a speaker, e.g., buzzer, vibrator or piezo element. The acoustic sensor may generate a discreet sound or vibration reminding the user to perform a planned task in accordance with information provided by, or via the software platform. The signal generated by the indicator may include a discreet sound, vibration, or the like.

In one embodiment of the invention, the smart device may comprise a housing securing the PCB. The housing may define one touch/push button for activation by the user. When the user activates the button by pressing/touching it, the touch sensor mounted on the PCB will therefore be activated and the smart device will generate the task signal. The button may occupy an entire surface of the housing. The housing may have different colours or patterns making it easier for the user to differentiate smart devices for different task registrations.

The smart device may be configured to be removably attach to an external object used by the user. The smart device may comprise a detachable attachment element, attached to the housing. The attachment element may include adhesive release liner, double adhesives, or similar elements. Alternatively, the smart device can be attached to an additional element which can then be attached to an object of interest, such as a tube, a tooth brush, a tablet pack, bottle hangers, keys, fridge or similar. The purpose is to place the smart device as close as possible to an object related to a task to be performed. For instance it can be a medication box, a lotion, or similar.

The software platform may be configured to map, to a task log, the task performed. The task log may be defined by the user via a user interface of the software platform running at the computer unit or it may be that a predefined task log is downloaded to the software platform through the computer unit. The user may create or download a plan that he wants to follow. At first, he may log in his own profile using credentials. Once the software platform is running, the user may connect one of the smart devices to the computer unit running the software and then create a plan with desired timing of the task. The task log may have a logo which is associated with the corresponding smart device, i.e. the logo may simply be of the same colour as the smart device, or it may have the same pattern as the smart device. The task log may define reminders, reminding the user to perform the task in case the user did not log the task as planned. Such reminders may be conveyed visually to the user through a display of the computer unit, acoustically through a loudspeaker of the computer unit and/or a loud speaker of the smart device, or by vibration of a vibrator of the computer unit, or by lighting the LED, or a built-in vibrator of the smart device. When the user performs the task, the task will be mapped to the task log upon the smart device activation.

The computer unit may comprise a memory element and may be configured to store the task log of one or more tasks, to compare the task log to the task signals received from the smart device and to produce an output signal in case the task signal does not match the task log. In one example, the task signal may not be timed well and in accordance to a schedule in the task log. Then the smart device may produce the output signal as an indication that the task is not performed as scheduled. The output signal may include a vibration of the smart device, or it may include a sound or lighting notification. In another example, the task signal may be registered too frequently by the user. The smart device or computer unit may generate a predefined output signal, different from other output signals, reminding the user not to perform the task as frequently as he does. Such a method may control the user to phase out bad habits or tasks which he is not supposed to perform.

The computer unit may display in a form of, e.g., a dashboard comprising a day, week and month, the schedule and the actual log of the smart device activations. The software platform may then indicate that the task was, e.g., 1) performed the right number of times but at the wrong schedule, 2) too many times compared to the schedule, 3) too few times compared to the schedule, or 4) the right number of times at the right schedule. A feedback system which may be implemented in the software platform, may inform the user about a status of the task. This may be performed via the smart device by means of vibrations or sound notification.

In case there is no plan, like for instance when the user wants to raise awareness on, e.g., cigarettes smoked, the smart device may simply be embodied as a tracking device logging the user's activities and displaying them on a dashboard of the computer unit.

Each smart device may comprise a wireless communication unit for establishing communication with the computer unit. It may include Bluetooth, infra-red wireless communication, Wi-Fi, zig-bee, etc. Wireless communication provides fast, easy, and well established standardized communication between devices, as well as low power consumption. Furthermore, the wireless connection unit may not influence the size and simplicity of the smart device.

The activity tracking system according to the present invention is preferably a system which can track, follow, plan, and/or review any routine that the user may perform. Furthermore, the system can track and review an activity that the user may want to avoid. A plan to be followed by the user may be downloaded into the memory medium of the smart device from the computer unit.

In the present context, the term task is to be interpreted as an action or a thing that has been performed or that is planned to be performed by the user. It can include any habit, use of a product, scheduled usage of a medicine, or the like. The task may relate to a wide variety of actions such as to skin treatments (such as treatment of psoriasis, eczema, dry skin, etc.), medication intake (birth control pills, blood pressure control pills, hormonal therapy, temporary therapy with antibiotics, etc.), healthy eating, chores (watering plants, vacuum cleaning, changing sheets, etc.) sport activities, meditation, social interaction, praying, gardening, studying, taking care of pets, personal hygiene, anxiety attacks, coffee intake, water intake, sleep, practicing music, reading, painting, working, practicing language, etc. Activities that the user may only want to track, but not schedule, may include stress, panic attacks, anger strikes, cigarette cravings, sexual desire, dating, drinking alcohol, nail-biting, spending money, etc.

The system is preferably capable of tracking one or more types of tasks and/or activations (i.e. activities), such as two, such as three, such as four, such as six, such as seven, and such as twelve. A larger number of different tasks may be tracked. The larger the number of the activities tracked is, the larger the complexity of the system is.

The user can be any person capable of using the computer unit, such as a person older than 3 years. Each user may have its own profile in the software platform, or multiple users may share one profile, the profile being distinguished by username and password. Likewise, in embodiments of the invention comprising a plurality of smart devices connected - or connectable - to the computer unit, each smart device associated with the computer unit may be distinguishable in a user interface of the software platform executing on the computer unit. The user may use the system for job related tasks, home activities, health related routines, gaming, and the like. The user may be a patient, a nurse or doctor, a worker at a construction site, a salesman in a store, etc.

The smart device may be placed or attached to any surface of interest such as a medication box, cosmetic product, cigarette pack, toothbrush glass, fruit bowl, fridge, mirror, yoga mat, plant pot, mobile phone, monitor, car dashboard, book, musical instrument, running shoes, necklace, bracelet, mirror, computer, desk, water bottle, wall, bicycle, gardening tool, cabinet, motorbike, key holder, telephone, and the like. For attaching the smart device to the surface of interest, an additional element may be used to ensure a firm attachment. It may, e.g., be an adhesive sticker, magnet, double adhesive or the like. The smart device may be detached from the surface at any times when required.

The software platform may be a mobile phone app or any other computer implemented programme. The software platform includes a log-in page, ensuring the user access to his/hers own profile, or to the shared profile. It may be programmed to, e.g., show a diagram representing a usage vs. a planned usage of a product.

The computer unit may be any computing device capable of running the software platform. It may include a mobile phone, a smart phone, a smart watch, a tablet, a PC, such as a laptop, a desktop computer, or similar. The computer unit preferably connects to and communicates to a back-end server system. The user can switch from using one computer unit to another, as all the registered or planned tasks are stored under the user's profile which is preferably stored at the back-end server system. The computer unit preferably comprises a communication platform enabling wireless connection to the one or more smart devices. Each smart device is preferably equipped with a compatible communication platform enabling the connection with the computer unit. When the connection between the computer unit and smart device is established, the software platform may exchange information between the two. Information exchanged may be related to the task performed, or the task which is to be performed, or simply to reminding the user of a scheduled task. Therefore, information which is exchanged can be either sent from the smart device to the computer unit or from the computer unit to the smart device, i.e., the communication may be bi-directional. In one example, the computer unit may download the plan or schedule to the smart device, so that the smart device can fire alarms when the connection to the computer is interrupted (computer unit out of range or tuned off).

In the present context, the task signal is to be interpreted as a signal to be sent by the smart device to the computer unit upon activation of the smart device by the user. The task signal is preferably sent via an established wireless connection between the smart device and the computer unit. When the user completes the task, he may activate the smart device and the smart device will then send the task signal. The smart device can be activated simply by touch, push, click, hold, move, voice or other sound generation, or in a similar manner. Typically, each smart device is preprogramed for one task only. However, it may also be that each type of activation relates to a different task.

The computer unit may then receive the task signal sent by the smart device, and the performed task may be logged in the software platform. The task signal may be received when wireless connection between the computer unit and the smart device is established. The task signal may carry a time stamp comprising time and date of the smart device activation as this time may differ from time when the computer unit receives the task signal.

In one embodiment of the invention, one smart device can be used for tracking more than one task. By programming the smart device for tracking multiple tasks, a cost of the system may be reduced and complexity of the hardware is minimized. Another advantage in having a single smart device tracking multiple tasks is that the smart unit may be kept in the most favourable location to act as a visual reminder. For instance, the smart device may be attached to a key chain, necklace, bracelet, fridge, etc.

The smart device may preferably be activated by different types of activation actions. Each activation type may determine a different task type, so that different tasks may be logged by different types of smart device activation. Thus, each task may be associated with a corresponding activation type. The activation type may be defined and assigned to the task through the software platform. For instance, a touch of the smart button may correspond to a usage of a first product, a short-duration click may correspond to cigarette intake, and a long-duration hold may correspond to tooth brushing.

The different task types may, e.g., include medication intake control, household duties, or behaviour control. Medication intake may relate either to a chronical disease treatment or to a temporary or an acute diagnosis. Household duties may include vacuum cleaning, cleaning out closets and dressers, washing windows, etc. Behavioural control may include cigarette intake, workout, eating habits, tooth brushing, etc.

Each smart device may have a unique identifier (UI). The smart device may advertise its UI for recognition by the computer unit once the connection between the smart device and the computer unit is established. The software platform may then save the UI of the smart device and assign it to the user's profile and to one or more tasks. By assigning one smart device to one user via the UI, accidental connection between the smart device and another computer unit may be avoided.

The software platform may be configured to identify the location of each smart device. Even if the smart device is out of the computer unit's reach, the location can be identified. This may help the user to find the smart device in case it is lost.

One smart device may be configured for use by multiple users. In this context, the smart device may be connected either to one computer unit or to multiple computer units. In case it is connected to one computer unit, multiple users may be all assigned to one profile. In one example, all the users may be registering the same task, such as household related duties. In another example each user may have different task assigned. The smart device may be configured to distinguish users by their finger prints and in that way differentiate which task is to be registered and by whom. In case the smart device is connected to multiple computer units, each user may define its own profile to which the smart device will be assigned.

The smart device may have a generally rectangular shape. It may not be wider than 3 cm, not longer than 3 cm, and not thicker than 1.5 cm, and/or it may have a diameter of at most 40 mm, such as at most 32 mm. It may have a substantially rectangular shape, being e.g. at most 2 cm wide, at most 2 cm long and at most 1.3 cm thick, such as at most 0.5 cm thick. Different dimensions of the smart device are also possible, and may depend on an application of the smart device. If the smart device is supposed to be attached to a larger surface it may have larger size, such as at most 3x3x1cm. In another example, it may be as small as 1x1x0.4 cm or less if it has to be attached to, e.g. mobile phone or the like.

In a second aspect, the present invention relates to a kit comprising a plurality of smart devices for use in the tracking system described above. Each one of the smart devices of the kit comprises:
- at least one interface for push, touch or voice activation by a user;
- a printed circuit board (PCB), the PCB comprising a memory medium configured to temporarily store at least one task signal generated upon activation of the button, the memory further storing a unique identifier (UI) of the smart device; and
wherein the smart device is further configured to output the unique identifier through wireless communication.

Each of the smart devices from the kit can connect to a computer unit. Once the smart device from the kit is turned on it may advertise its UI and attempt to establish a connection with a nearby computer unit running the software platform. The smart devices of the kit may be used in all the scenarios described above and in relation to the first aspect of the invention.

In the third aspect, the present invention relates to a method for tracking a user's activity. The method comprises the steps of:
- providing one or more smart devices, a computer unit and a software platform executable on the computer unit, wherein each smart device comprises a printed circuit board (PCB), a memory medium, and an interface for push, touch or voice activation by a user;
- pairing each one of the smart devices with the computer unit;
- pairing a first smart device with the computer unit by establishing a wireless connection between the two and by communicating a first unique identifier of the smart device from the smart device to the computer unit, when the first smart device is within reach of the computer unit, and capturing the first unique identifier in the software platform at the computer unit;
- subsequently pairing a second smart device with the computer unit by repeating the step of establishing a wireless connection between the two, and by communicating a second unique identifier of the second smart device from said second smart device to the computer unit, and capturing the second unique identifier in the software platform at the computer unit;
- defining, in the software platform, respective tasks associated with each one of the smart devices;
- generating one or more task signals at the smart devices upon activation thereof by a user;
- upon the activation, transmitting to the computer unit one or more task signals generated by the smart devices.

The method may further comprise the steps of storing one or more task signals in the memory of the smart devices when no connection to the computer unit is available until a wireless connection to the computer unit becomes available; and automatically transmitting the task signals to the computer unit when the wireless connection to the computer unit has become available.

### BRIEF DESCRIPTION OF THE DRAWINGS

Embodiments of the invention will now be described with reference to the drawings, wherein:
- Fig. 1: illustrates a first embodiment of an activity tracking system.
- Fig. 2: illustrates a second embodiment of the activity tracking system.
- Fig. 3: illustrates authentication procedure in the first embodiment of the activity tracking system.
- Fig. 4: illustrates an exploded view of a smart device.
- Fig. 5: illustrates a flowchart related to a method for tracking a user's activity.

### DETAILED DESCRPTION OF THE DRAWINGS

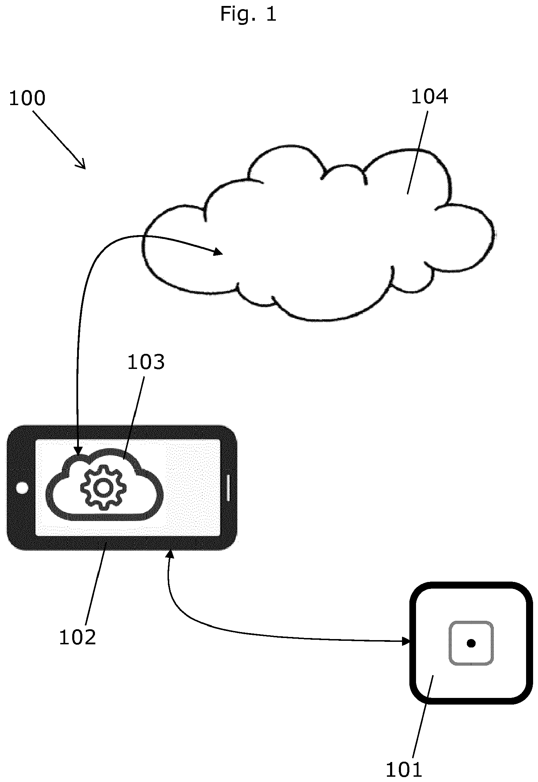

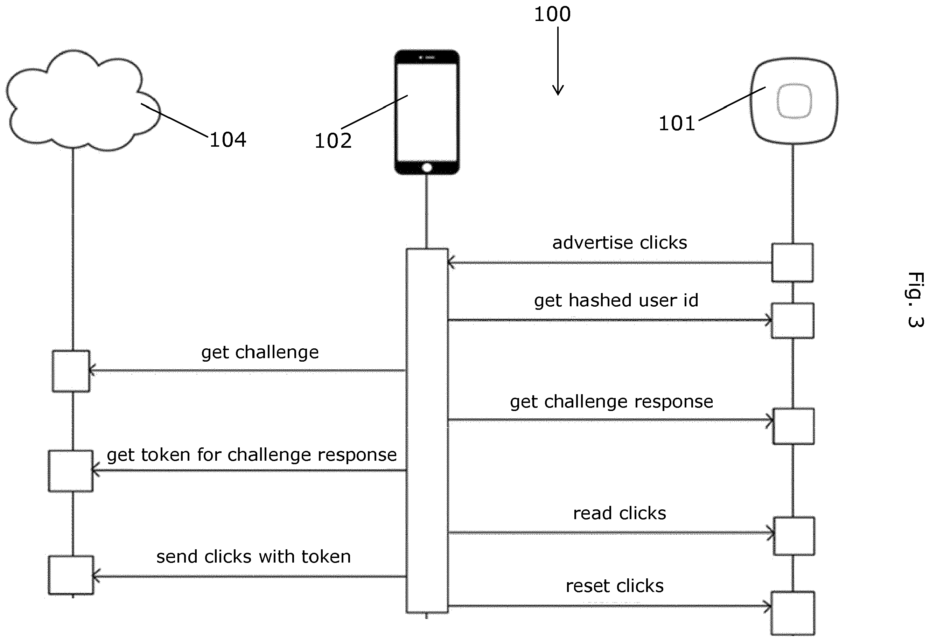

Fig. 1 illustrates a first embodiment 100 of an activity tracking system. The activity tracking system 100 comprises a smart device 101, a computer unit 102, and a software platform 103. The computer unit 102 is wirelessly connected to the smart device 101. The computer unit 102 is configured to run the software platform 103. The software platform 103 is configured for exchanging information between the smart device 101 and the computer unit 102. Information exchange is managed by an authentication procedure as it will be described in more details in relation to Fig.3. The computer unit 102 is in communication with a server of a backend server system being part of an Internet cloud 104. The server in the cloud 104 keeps all the user accounts, their task logs, and each activation of any smart device 101. The smart device 101 may be activated by one or more users. It may also serve for registering and tracking of one or more tasks.

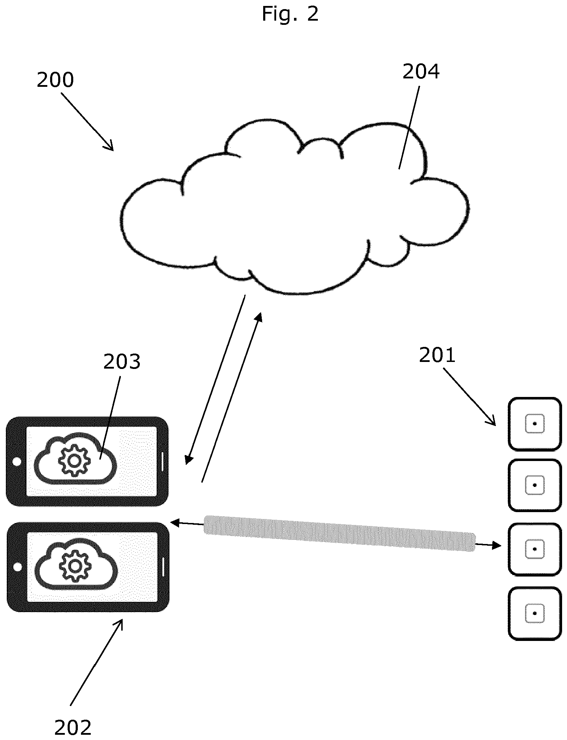

Fig. 2 illustrates a second embodiment 200 of the activity tracking system. In this embodiment, the activity tracking system 200 comprises four smart devices 201 and two computer units 202, each running the same software platform 203. Communication between the smart devices 201 and the computer units 202 may be set up in a number of different ways. In one example, one of the smart devices 201 may be wirelessly connected to one of the computer units 202 while three other smart devices 201 may be communicating with the other computer unit 202. In the later example, typically more than one user will be involved as each of the two computer units 202 may be connected to one user account. As in the first embodiment described in Fig. 1, the computer units 202 are communicating with the server being part of the Internet cloud 204.

Fig. 3 illustrates an authentication procedure for the first embodiment of the activity tracking system 100. The same procedure is applicable in any other configuration of the system. Once the smart device 101 is turned on and activated by the user it starts advertising activation clicks to the computer unit 102. The computer unit 102 will then acquire, from the smart device 102 a hashed user's ID and then communicate the user's credentials to a server in an Internet cloud 104. The server stores all the established credentials of different users. If the server recognizes the user's username and password, the computer unit 102 will approve the smart device by sending a challenge response to it as well as sending a token for challenge response to the server in the cloud 104. The system is then ready to generate and process the user's tasks. Once the user has perform a task, he will click the smart device and these will be sent to the computer unit. Once the click is received by the computer unit, the computer unit will send, to the server in the cloud 104, the click with the token. In this way the task will be associated to the user's account, and will be mapped to a possibly existing plan made by the user.

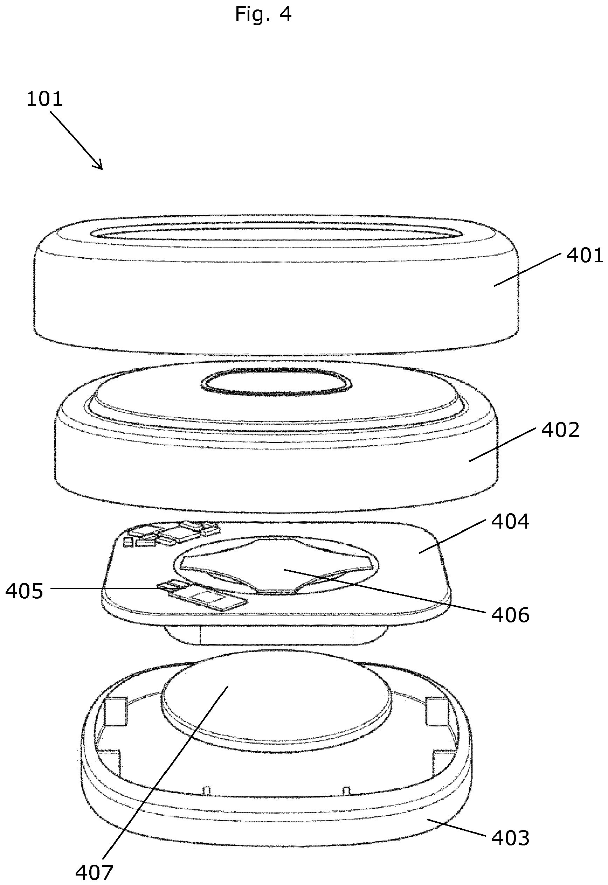

Fig. 4 illustrates an exploded view of a smart device 101. A housing of the smart device 101 may comprise an exchangeable colour ring 401 which identifies each smart device at a glance. The ring 401 may be fitted to a textured PP plastic top part 402 of the housing, designed for optimal tactility and pleasurable to touch. The top part 402 of the housing is then fitted to a bottom part 403 of the housing. In alternative embodiments (not shown), the devices comprises voice recognition means or a touch interface. The top part may also have a house port to let sound out, or a light guide port to let LED light out. The bottom part 403 may also be made of the textured PP plastic. A replaceable adhesive on the back of the bottom part 403 may be attached, to stick the smart device 101 to any surface of relevance, a product that the user wants to use, or similar. Inside the housing is a PCB 404 which comprises necessary electronics for proper functioning of the smart device 101. The PCB 404 may comprise LED lights 405 which may light upon every activation of the smart device 101. Further, the PCB 404 may comprise a switch, such as a high sensitivity dome switch 406, which senses the activation of the smart device 101. The smart device 101 comprises a high capacity built-in battery 407 for extended operation. The PCB may also comprise sound/vibration elements such as buzzers, piezo elements, vibrators, speakers, and/or elements for motion detection, push, touch and voice activation, such as touch capacitors, and the like.

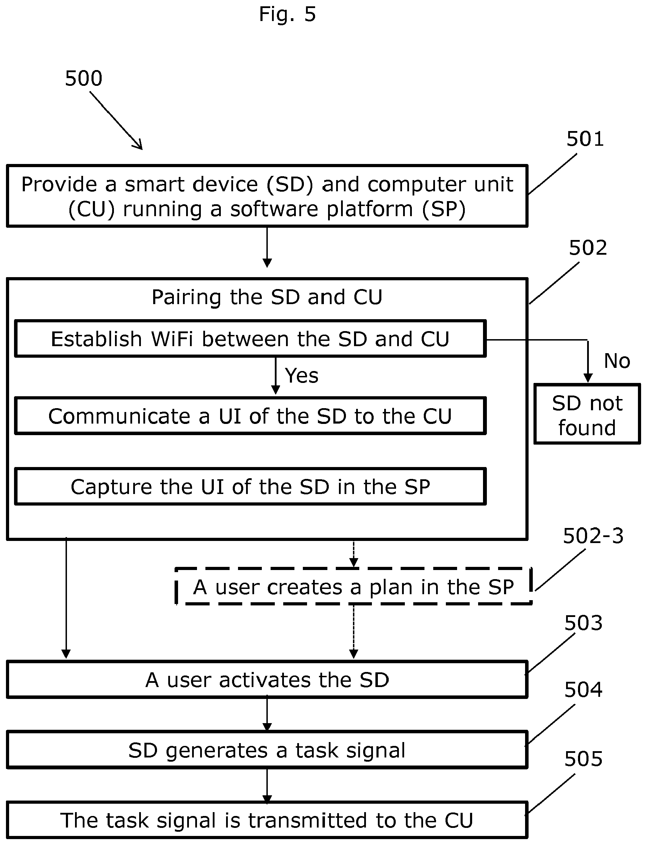

Fig. 5 illustrates a flowchart 500 related to a method for tracking a user's activity. To track a user's activity, the first step 501 is to provide one or more smart devices (SDs) along with a computer unit (CU) and a software platform (SP) executable on the computer unit. Each smart device may comprise a PCB, a memory medium, and an interface for user activation, such as a push button for finger activation by a user, and/or an interface for touch and/or voice activation. Once the smart devices and computer unit are provided, a step 502 of pairing each one of the smart devices with the computer unit is to be performed. Paring is performed by establishing a wireless connection (WiFi) between a first smart device and computer unit and by communicating a first UI of the first smart device from the first smart device to the computer unit. It is required that the first smart device is within reach of the computer unit. Then the first UI will be captured in the software platform at the computer unit. If there is no connection between the first smart device and computer unit, UI cannot be communicated, and the software platform will show a message on the computer unit that the smart device cannot be found. Subsequently, the second, and the third, etc. smart device is paired in the same manner, i.e., establishing a wireless connection between the smart device in the queue, and by communicating a corresponding UI of the smart device from said smart device to the computer unit, and capturing the unique identifier in the software platform at the computer unit. In some cases, the computer unit may establish communication with only one smart device, and smart communicating with it, and only later start establishing communication with other smart devices being part of the system. Thus, there may be no strict rule on the order of connecting. It may also be the user who triggers the communication between the computer unit and one of the smart devices. The software platform may have a built-in option for the user to search for the smart devices in proximity of the computer unit. The smart devices may be able of establishing the wireless connection if they are not further than a predetermined distance from the computer unit. In respect of BlueTooth connections, the predetermined distance may be 50 m, whereas in respect of other types of connections it may be more.

Once the connection between the computer unit and one or more smart devices is established, the system is ready to be used by the user. The user is to activate one of more of the smart devices, by pressing the button of the smart device, step 503. The smart device will then generate a task signal, step 504. The task signal is then transmitted from the smart device to the computer unit, step 505. In case of multiple smart devices, multiple task signals are sent.

Optionally, the user may, in the software platform, define respective tasks associated with each one of the smart devices, step 502-3. He may create a plan to be followed, and track the planned activities by registering then by activation of the smart device.

## Claims

1. An activity tracking system configured to track one or more tasks performed by a user, the system comprising at least one smart device, a software platform, and a computer unit wirelessly connected to the at least one smart device, the computer unit being configured to run the software platform, the software platform configured for exchanging information between the at least one smart device and the computer unit,
wherein each smart device is configured to send, to the computer unit, a task signal related to a task performed by the user, the task signal being generated when the user activates the smart device by push, touch, voice activation, or by movement thereof and wherein the computer unit is configured to receive the task signal and log the task performed along with time and date of the task,
and wherein the smart device comprises a printed circuit board (PCB), the PCB comprising a memory medium configured to temporarily store at least one task signal along with time and date of the task in case the computer unit is out of reach.

2. An activity tracking system according to claim 1, wherein the smart device comprises a housing securing the PCB, the housing defining one touch/push button for activation by the user.

3. An activity tracking system according to claim 1 or 2, wherein the smart device is configured to be removably detachably attached to an external object used by the user.

4. An activity tracking system according to any or the previous claims, wherein the software platform is configured to map the task performed to a task log, the task log being at least partially defined by the user and/or at least partially downloaded from a remote entity.

5. An activity tracking system according claim 4, wherein the computer unit is configured to store the task log of one or more tasks, to compare the task log to the task signals received from the smart device and to produce an output signal in case the task signal does not match the task log.

6. An activity tracking system according to any or the previous claims, wherein each smart device comprises a wireless communication unit for establishing communication with the computer unit.

7. An activity tracking system according to any or the previous claims, wherein one smart device is used for tracking more than one task.

8. An activity tracking system according to any or the previous claims, wherein the smart device is activated by different types of activation clicks, each click type determining a different task type.

9. An activity tracking system according to any or the previous claims, wherein each smart device has a unique identifier.

10. An activity tracking system according to any or the previous claims, wherein the software platform is configured to identify the location of each smart device.

11. An activity tracking system according to any or the previous claims, wherein one smart device is configured for use by multiple users, the smart device being configured to distinguish users by their finger prints.

12. An activity tracking system according to any or the previous claims, wherein the smart device has a generally rectangular shape, the smart device being not wider than 4 cm, not longer than 4 cm, and not ticker than 1.5 cm.

13. A kit comprising a plurality of smart devices for use in a system according to any of the preceding claims, wherein each one of the smart devices comprises:
- at least one input interface for push, touch, voice or movement activation by a user;
- a printed circuit board (PCB), the PCB comprising a memory medium configured to temporarily store at least one task signal generated upon activation of the button, the memory further storing a unique identifier of the smart device;
and wherein the smart device is further configured to output the unique identifier through wireless communication.

14. A method for tracking a user's activity, the method comprising the steps of:
- providing one or more smart devices, a computer unit and a software platform executable on the computer unit, wherein each smart device comprises a printed circuit board (PCB), a memory medium, and an interface for motion detection, push, touch or voice activation by a user;
- pairing each one of the smart devices with the computer unit;
- pairing a first smart device with the computer unit by establishing a wireless connection between the two and by communicating a first unique identifier (UI) of the smart device from the smart device to the computer unit, when the first smart device is within reach of the computer unit, and capturing the first unique identifier in the software platform at the computer unit;
- subsequently pairing a second smart device with the computer unit by repeating the step of establishing a wireless connection between the two, and by communicating a second unique identifier of the second smart device from said second smart device to the computer unit, and capturing the second unique identifier in the software platform at the computer unit;
- generating one or more task signals at the smart devices upon activation thereof by a user;
- upon the activation, transmitting to the computer unit one or more task signals generated by the smart devices.

15. A method according to claim 14, the method further comprising the steps of:
- defining, in the software platform, respective tasks associated with each one of the smart devices;
- storing one or more task signals in the memory of the smart devices when no connection to the computer unit is available until a wireless connection to the computer unit becomes available; and
- automatically transmitting the task signals to the computer unit when the wireless connection to the computer unit has become available.
